# EUROPEAN PATENT SPECIFICATION

(11) **EP 2 488 165 B1**
(45) Date of publication and mention of the grant of the patent: **23.07.2014**
(21) Application number: 10771208.5
(22) Date of filing: 12.10.2010
(51) Int. Cl.: A61K 9/51, A61K 9/00, A61K 48/00, C12N 15/11, A61P 21/00

(54) **NANOPARTICLE OF THE CORE-SHELL TYPE SUITABLE FOR DELIVERING THERAPEUTIC OLIGONUCLEOTIDES TO TARGET TISSUES AND THE USE THEREOF FOR THE PREPARATION OF A MEDICAMENT FOR TREATING DUCHENNE MUSCULAR DYSTROPHY**
KERN-HÜLLE-NANOPARTIKEL ZUR FREISETZUNG VON AUF GEWEBE GERICHTETEN THERAPEUTISCHEN OLIGONUKLEOTIDEN UND IHRE VERWENDUNG ZUR HERSTELLUNG EINES ARZNEIMITTELS FÜR DIE BEHANDLUNG VON DUCHENNE-MUSKELDYSTROPHIE
NANOPARTICULE DU TYPE NOYAU-COQUE APPROPRIÉE POUR L'ADMINISTRATION D'OLIGONUCLÉOTIDES THÉRAPEUTIQUES À DES TISSUS CIBLES ET UTILISATION DE CELLE-CI POUR LA PRÉPARATION D'UN MÉDICAMENT POUR TRAITER UNE DYSTROPHIE MUSCULAIRE DE DUCHENNE

(30) Priority: 14.10.2009 IT TO20090782
(43) Date of publication of application: 22.08.2012
(73) Proprietor: Universita' degli Studi di Ferrara, 44100 Ferrara (IT)
(72) Inventor: FERLINI, Alessandra, 44121 Ferrara (IT); MEDICI , Alessandro, 40138 Bologna (IT); PERRONE , Daniela, 44124 Porotto (Ferrara) (IT); RIMESSI , Paola, 44121 Ferrara (IT); TONDELLI , Luisa, 40122 Bologna (IT); LAUS , Michele, 15100 Alessandria (IT); SPARNACCI , Katia, 47853 Coriano (Rimini) (IT)
(74) Representative: Comoglio, Elena
(86) International application number: PCT/IB2010/054618
(87) International publication number: WO 2011/045747

(56) References cited:
- WO-A1-2005/048997

## Description

The present invention relates to a nanoparticle of the core-shell type effective in delivering therapeutic oligonucleotides into target tissues and in particular into muscle cells. The invention further relates to a therapeutic nanoparticle/oligonucleotide complex designed to be used as an active principle in a medicament, particularly a medicament for the therapeutic treatment of Duchenne muscular dystrophy. In the context of the present description, the term "nanoparticle" indicates that the average diameter of a test particle sample is comprised between about 180 nm and 200 nm.

Duchenne muscular dystrophy (DMD) is a degenerative X chromosome-linked hereditary muscular disease, mainly caused by mutations that induce disruption of the reading frame, due to extensive rearrangements in the Dystrophin gene. DMD patients are affected by a serious degeneration of the skeletal muscle and by a dilatative cardiomyopathy.

The minor allelic form of the disease, designated as Becker muscular dystrophy, is caused by mutations that do not alter the reading frame and allow for the synthesis of a shorter yet functional protein.

Recently, new therapeutic approaches for DMD have been proposed, some of which have been tested so far only in animal models. Others, such as for example the use of drugs (PTC124) designed to induce reversion of the stop codon and exon skipping mediated by antisense oligonucleotides (AON), have already been put into practice as clinical trials. Such novel therapeutic options represent a first step towards a potential cure for this devastating disease.

However, numerous obstacles are still to be overcome in the therapeutic treatment of Duchenne muscular dystrophy through antisense oligonucleotides. Among which, there may be particularly mentioned the need to optimise the non-toxic effective doses, the improvement of the delivering system for the drug so as to favour the reaching of all the affected tissues, the finding of a simple administration mode, which is essential for treatments bound to last throughout the life of the patient.

Rimessi P. et al. Mol Ther. May 2009; 17(5):820-7, demonstrated that inert PMMA nanoparticles, designated as T1, conjugated with antisense oligoribonucleotides (AON) are capable of inducing restoration of the dystrophin protein in the *mdx* animal model. However, the effectiveness of such a system is relatively unsatisfactory.

WO 2005/048997 describes nanoparticles for delivery of a pharmacologically active agent.

In order to obtain a system that is more efficient for delivering therapeutic oligonucleotides into target tissues, particularly muscle tissue, the present inventors have designed and prepared a new type of core-shell nanoparticle, designated as ZM2.

One first aspect of the invention is therefore a nanoparticle of the core-shell type which comprises:
(i) a core consisting essentially of poly(methyl methacrylate) (PMMA), and
(ii) a shell consisting of a plurality of chains of a random copolymer of units of N-isopropyl-acrylamide and units of a reactive methacrylate bearing cationic groups.

In a preferred embodiment, the aforesaid reactive methacrylate units bearing cationic groups have the following structural formula: 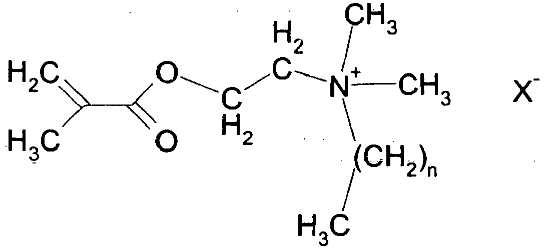 wherein n is an integer comprised between 1 and 10, preferably 7, and X⁻ is a counter ion, preferably selected from bromide, chloride and fluoride; the bromide ion being the most preferred among them.

The nanoparticles of the invention are designated in short as "ZM2 nanoparticles".

The ZM2 nanoparticles have the feature of easily giving rise to a reversible association with oligonucleotide molecules, through absorption of one or more negative-charge oligonucleotide molecules onto or into the outer shell of the nanoparticle, which by contrast is charged positively.

Therefore; a further aspect of the present invention is a nanoparticle/oligonucleotide complex comprising a ZM2 nanoparticle of the invention and one or more copies of an oligonucleotide molecule associated therewith.

The nanoparticle/oligonucleotide complex of the invention is particularly suitable as a system for delivering therapeutic oligonucleotides into target cells, since such a complex easily enters target cells and does not show cytotoxicity effects neither *in vitro* nor *in vivo.* Therefore, the nanoparticle/oligonucleotide complex of the present invention is particularly suitable for transfecting therapeutic oligonucleotides into target eukaryotic cells, preferably muscle cells (smooth or striated muscle), for the treatment of Duchenne muscular dystrophy.

In a preferred embodiment, the therapeutic oligonucleotide is an antisense oligoribonucleotide.(AON) capable of restoring, in all or in part, the expression of a functional dystrophin protein within muscle cells by the exon skipping mechanism, and thus designed to be used for the therapeutic treatment of Duchenne muscular dystrophy.

The tests carried out by the present inventors demonstrated that the ZM2 nanoparticles of the invention are not cytotoxic neither *in vitro* nor *in vivo,* even after repeated administrations.

Therefore, another aspect of the present invention is the use of the therapeutic nanoparticle/oligonucleotide complex as defined above for the preparation of a medicament for the therapeutic treatment of Duchenne muscular dystrophy. To this end, a particularly preferred oligonucleotide is the antisense RNA oligoribonucleotide modified with 2'-O-methyl phosphorothioate (20MePS) having the nucleotide sequence 5'-GGCCAAACCUCGGCUUACCUGAAAU-3' (SEQ ID NO:1), designated in short as "M23D". The medicament for the treatment of Duchenne muscular dystrophy is prepared for instance as a pharmaceutical formulation suitable for administration by injection, such as an injectable liquid solution or suspension. A pharmaceutical formulation typically comprises, besides the specific active principle required for the therapeutic effect, pharmaceutically acceptable excipients, carriers and/or diluents optionally useful to the formulation, the selection and correct use of which are within the skills of persons of ordinary skill in the art.

In order to prove the therapeutic effectiveness of the therapeutic nanoparticle/oligonucleotide complex according to the invention, the present inventors synthesized the ZM2 nanoparticle according to the procedures described in Example 1 and loaded the ZM2 nanoparticles thus obtained with the 2'-O-methyl phosphorothioate-modified RNA oligonucleotide M23D (SEQ ID NO:1) as described in Example 2, thereby obtaining a ZM2/AON(M23D) complex. Such a complex was administered by the intra-peritoneal route to *mdx* mice (an animal model for Duchenne muscular dystrophy) to assess the restoration of the synthesis of a functional dystrophin and the localization thereof within muscle cells. The experiments performed *in vivo* in the *mdx* mouse are described in detail in the examples that follow.

The tests carried out by the present inventors demonstrated that the ZM2/AON(M23D) complex of the invention is capable of inducing at a high efficacy the restoration of dystrophin in the quadriceps, gastrocnemius and diaphragm skeletal muscles, as well as the cardiac muscle, as assessed by immunofluorescence analysis. A real time exon-specific RT-PCR (ESRA) was also carried out to calculate the percentage of skipping of exon 23 in the skeletal muscle and cardiac muscle, by analysis of the RNA transcript purified from the different tissues. It has thus been found that in the skeletal muscles the percentage of skipping of exon 23 is twice as that achieved by treatment of *mdx* mice with the T1/AON(M23D) complex of the prior art. Also by Western blotting, the presence of high molecular weight dystrophin was shown in the skeletal muscles (quadriceps and diaphragm) of *mdx* mice treated with ZM2/AON(M23D). It is particularly interesting to note that the total amount of AON(M23D) injected in the form of a complex with the ZM2 nanoparticles of the invention (dose of 52.2 mg/kg of body weight proven effective) resulted considerably lower than the values reported in the previous studies concerning the systemic administration of naked AON (120-600 mg/kg of body weight).

The obtained experimental evidence indicates that the novel ZM2 nanoparticle/oligonucleotide complex of the invention increases considerably the restoration of dystrophin in the skeletal muscles of *mdx* mice, being also slightly effective in the cardiac muscle, which allows to confirm that the ZM2. nanoparticles represent an extremely promising carrier for the systemic delivering of therapeutic oligonucleotides. Restoration of dystrophin was proven both at the RNA and protein level: (i) a percentage of skipping of 10-20%, particularly surprising considering that the dose of oligonucleotide (AON) administered is instead very low, and (ii) expression of high molecular weight dystrophin. The new dystrophin localises correctly at the sarcolemma in 40% of the fibres, as detected by immunohistochemical analyses.

Moreover, the ZM2 nanoparticles of the invention were observed to advantageously combine a slow release and depot effects (storage) of the oligonucleotides associated therewith. These phenomena, together with the protection from degradation and the high binding ability, make the ZM2 nanoparticles of the invention particularly efficient and effective as carrier systems for drugs.

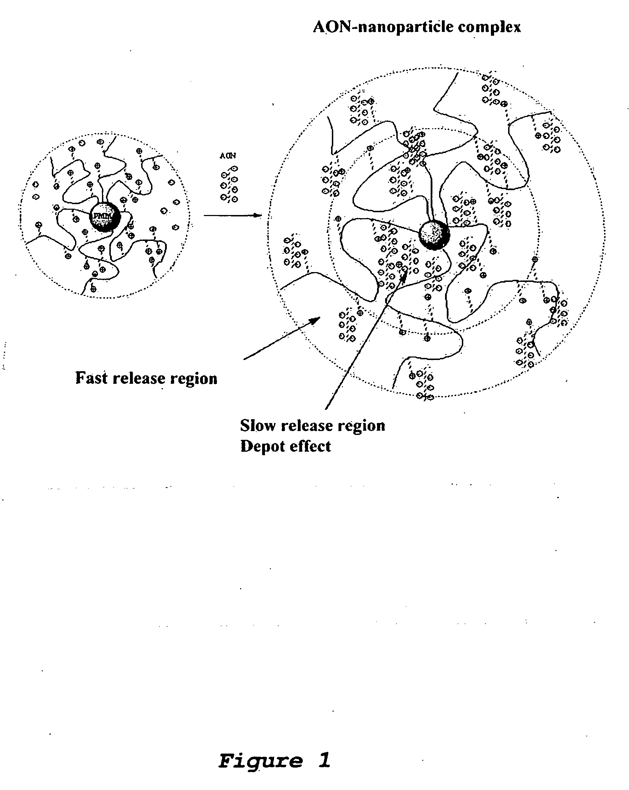

Figure 1 is a schematic representation of the structure of the ZM2 nanoparticles (137 nm average diameter), of their interaction with the oligonucleotides and of the fast and slow release regions, which account for the depot effect.

In short, the obtained results demonstrate that the ZM2 nanoparticle/oligonucleotide complex of the invention administered by the systemic route is effective in restoring the synthesis of dystrophin in the striated muscles at low doses of oligonucleotide.

The following examples are provided merely by way of illustration and not as a limitation of the scope of the invention, as defined in the appended claims. The independent and dependent appended claims are an integral part of the description.

### EXAMPLES

### Example 1: synthesis of the ZM2 nanoparticles

### Materials and methods

2-(Dimethylamino)ethyl methacrylate (DMAEMA) (comonomer 1), 1-bromooctane, N-isopropylacrylamide (comonomer 2) and 2,2'-azobis(2-methylpropionamidine) dihydrochloride (AIBA) were purchased from Aldrich. All these products were used without further purification. Methyl methacrylate (MMA) was purchased from Aldrich and distilled under vacuum immediately prior to use.

The potentiometric titrations were performed with a benchtop CyberScan pH 1000 pH meter equipped with an ATC probe and an Ingold Ag 4805-S7/120 silver electrode. The quantity of the quaternary ammonium group per gram of nanoparticle was determined by potentiometric titration of the bromide ions obtained after complete ion exchange. The ion exchange was accomplished by dispersing 0.5 g of a nanoparticle sample in a beaker into 25 ml of 1M KNO₃ at room temperature for 48 hours. Under these conditions, a quantitative ion exchange was achieved. The mixture was then adjusted to pH = 2 with dilute H₂SO₄ and the bromide ions in solution were titrated with a 0.01 M AgNO₃ solution.

The size of the nanbparticle was measured by a JEOL JSM-35CF scanning electron microscope (SEM) with an accelerating voltage of 20 kV.

The samples were coated by vacuum metallization with a thin layer (10-30 A) of gold. The magnification is given by the scale in each micrograph. The SEM photographs were digitalized, using the Kodak photo-CD system, and processed with the NIH Image (version 1.55, in the public domain) image processing program. From 150 to 200 individual nanoparticle diameters were measured for each optical micrograph.

The Z-average particle size and the poly-dispersion index (IP) were determined by dynamic light scattering at 25°C with a Zetasizer 3000 HS system (Malvern, United Kingdom) using a 10 mV He-Ne laser and a PCS software for Windows (version 1.34, Malvern, United Kingdom). For data analysis, the viscosity and refractive index of purified water at 25°C were used. The instrument was subjected to inspection with standard polystyrene latex having a 200-nm diameter.

### synthesis of the ionic comonomer (1)

The ionic (comonomer 2-(dimethyloctyl)ammonium ethyl methacrylate bromide (1) was obtained by direct reaction of DMAEMA with 1-bromooctane. DMAEMA (0.166 mol) was mixed with 1-bromooctane (0.083 mol) without any additional solvent. After adding a small portion of hydroquinone to inhibit the radical polymerization reactions, the mixture was stirred at 50°C for 24 hours. The solid product thus obtained was washed with dry diethyl ether to remove excess DMAEMA. Finally, the product was dried under vacuum at room temperature. The purity of the product was tested by ¹H NMR. The reaction yields were in the range from 55 to 65%.

### Preparation of the ZM2 nanoparticles

In a typical polymerization reaction by emulsion, 30 ml (280 mmol) of methyl methacrylate were introduced dropwise into a 500 ml round-bottom flask containing an aqueous solution of 3.50 g (10 mmol) of the ionic comonomer (1) obtained as described above and 1.13 g (10 mmol) of the non-ionic comonomer (2). The flask was fluxed with nitrogen under continuous stirring for 30 minutes, then 85 mg (0.313 mmol) of the cationic free radical initiator AIBA, dissolved in water, were added.

The flask was fluxed with nitrogen throughout the polymerization, which was performed at 80 ± 1.0°C for 2 hours under constant stirring. At the end of the reaction, the product was filtered and purified by repeated dialysis, at least 10 times, against an aqueous cetyl trimethyl ammonium bromide solution, to remove the residual methyl methacrylate, and then water, at least 10 times, to remove the residual comonomer. The nanoparticle yield, compared to the total amounts of methyl methacrylate and water-soluble comonomers, was 60%. The nanoparticle sample presented an average diameter (as determined by photon correlation spectroscopy) of 190 nm. The quantity of comonomer units per gram of nanoparticles was estimated by potentiometric titration of the bromine ions obtained after complete ion exchange, and was found to be equal to 292 µmol/g.

### Example 2: determination of antisense oligoribonucleotide (AON) adsorption onto the ZM2 nanoparticles of the invention

The adsorption experiments were carried out by using the 2'-O-methyl phosphorothioate-modified RNA oligonucleotide M23D (SEQ ID NO:1). 1 mg of freeze-dried nanoparticles was suspended in 1 ml of 20 mM sodium phosphate buffer (pH 7.4), and sonicated for 15 minutes. A certain amount of a concentrated aqueous AON (M23D) solution was then added such as to obtain a final concentration of 10÷100 µg/ml. Experiments were carried out in triplicate (standard deviation ≤ 10%). The suspensions were continuously stirred at 25°C for 2 hours. A quantitative sedimentation of the nanoparticle/AON (M23D) complexes was obtained by microfuge centrifugation at about 20,000 rpm for 15 minutes. Aliquots (10÷50 µl) of the supernatant were drawn, filtered on a Millex GV4 filter unit and diluted with sodium phosphate buffer. Finally, UV absorbance at λ = 260 was measured. Adsorption efficiency (%) was calculated through the formula: 100 x (AON used)-(unbound AON)/(AON used).

The loading experiments showed that the ZM2 nanoparticles of the invention (1 mg/ml) adsorbed onto their surface the 2'OMePS-modified antisense oligoribonucleotide M23D in the concentration range from 10 to 100 µg/ml. 2'OMePS M23D adsorption onto the ZM2 nanoparticles was a highly reproducible process with a loading value of 90 µg/mg.

### Example 3: cytotoxicity analysis of the ZM2 nanoparticles

Monolayer cultures of HeLa cells were grown in DMEM medium (Gibco, Grand Island, New York; United States of America) containing 10% heat-inactivated FBS (Hyclone, Logan, UT, United States of America). The cells (1 ×10⁴/100 µl) were seeded in 96-well plates and cultured for 96 hours at 37°C with increasing concentrations of ZM2 nanoparticles (0.01-1 mg/ml) (6 replica wells). Cell proliferation was measured using the colorimetric cell proliferation kit I (based on MTT) (Roche, Milan, Italy), and compared to that of untreated cells. The experiments were repeated 3 times (S.D. ≤ 13%).

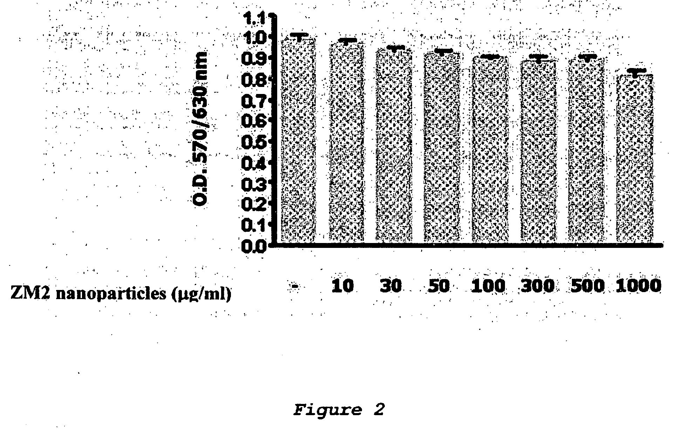

The cytotoxicity of the ZM2 nanoparticle sample was assayed in HeLa cells following incubation with increasing amounts of nanoparticles. As shown in Figure 2, no reduction in cell viability (p > 0.05) was observed at up to 1 mg/ml of ZM2, as compared to untreated cells. Although a slight reduction (10%) in cell viability was observed at very high doses of ZM2 (1 mg/ml), the difference was not statistically significant (p > 0.05), as compared to untreated cells. These results indicate that the ZM2 nanoparticles of the invention are non-toxic for the cells.

### Example 4: in vivo experiments in the mdx model

6-Week *mdx* mice were divided into 4 groups, which received the following treatments:
- group 1 (4 animals): intra-peritoneal injection of 225 µg of naked AON(M23D);
- group 2 (4 animals): intra-peritoneal injection of 225 µg of AON(M23D) conjugated with 2.5 mg of ZM2;
- group 3 (4 animals): intra-peritoneal injection of 2.5 mg of ZM2 in fresh sterile saline (0.9% sodium chloride); and
- group 4 (4 control animals): no intra-peritoneal injection.

The animals received treatment once a week for 7 weeks (7.5 mg/kg/injection) and were sacrificed 1 week after the last administration. The total amount of AON(M23D) administered to each animal was 52.5 mg/kg (1575 µg/animal).

1 Week after the seventh injection, 4 *mdx* mice from group 1 and 4 mice from group 2 were sacrificed; quadriceps, diaphragm, gastrocnemius and heart were isolated, dried, deprived of the external tendons, snap-frozen in isopentane cooled with liquid nitrogen and stored at -80°C until further use. 4 untreated *mdx* mice (group 4) and 4 *mdx* mice treated only with ZM2 nanoparticles (group 3) were also sacrificed as controls.

### Immunohistochemical assays

All immunohistochemical procedures were carried out as described in Rimessi P. et al. Mol Ther. May 2009; 17(5):820-7. Epub February 24, 2009.

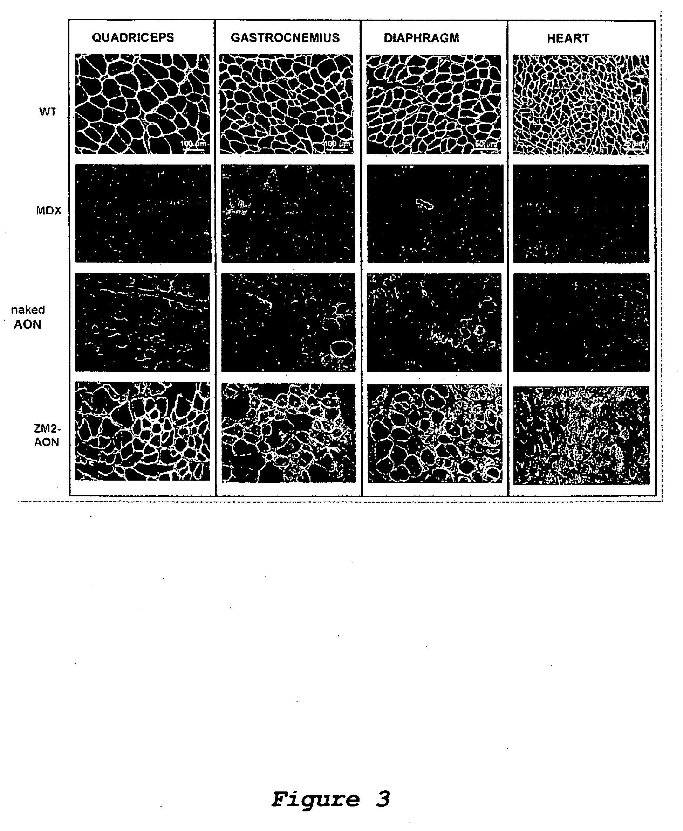

It can be seen from Figure 3 that dystrophin appears to be expressed correctly on the membrane of muscle fibres from *mdx* mice treated with ZM2/AON (M23D). The staining intensity and localization in the muscle fibres in which dystrophin-positivity was restored are reminiscent of those of the normal skeletal muscle. No dystrophin staining was visible at the sarcolemma of either untreated *mdx* mice or *mdx* mice treated only with the ZM2 nanoparticles.

Frozen cross-sections of the quadriceps skeletal muscle having a thickness of 7 µm obtained from untreated *mdx* mice (group 4), *mdx* mice treated with naked AON(M23D) (group 1), *mdx* mice treated with ZM2/AON(M23D) (group 3) and wild-type (WT) mice, were subjected to double staining with a rat monoclonal antibody against laminin-2 (α-2 chain, 4H8-2 Alexis Biochemical) and a rabbit anti-dystrophin polyclonal antibody (H-300, Santa Cruz Biotechnology), and were detected with a FITC-conjugated anti-rat or TRITC-conjugated anti-rabbit (DAKO) secondary antibody, respectively.

As the count of the positive fibres is considered as an essential parameter for assessing the effectiveness of dystrophin restoration, two independent methods were compared to allow for a more precise quantification of the dystrophin-positivity in muscles.

The first method is a manual system, the AnalySIS program (Soft Imaging System, Münster, Germany), which counts the dystrophin-positive fibres with a staining that covers at least 50% of the sarcolemma perimeter.

The second method is a novel semi-automatic and semi-quantitative analysis system that calculates the ratio of the dystrophin-positive areas to the laminin α-2 chain-positive areas in the double-stained samples, using a software developed in collaboration with Nikon Italia (NIS-Elements 3.0 AR imaging program, Nikon). After subtraction of the background, the average ratio of the dystrophin-coloured area to the laminin α2-coloured area (FITC/TRITC) was calculated. The laminin α-2 chain was chosen as a marker for basal laminin of muscle fibres. In order to provide a graphical representation of the aforesaid, the average values of the ratio between the FITC/TRITC areas in treated and untreated *mdx* mice was normalised against the WT values and expressed as a percentage of dystrophin-positive fibres over the total number of fibres (laminin α2-positive).

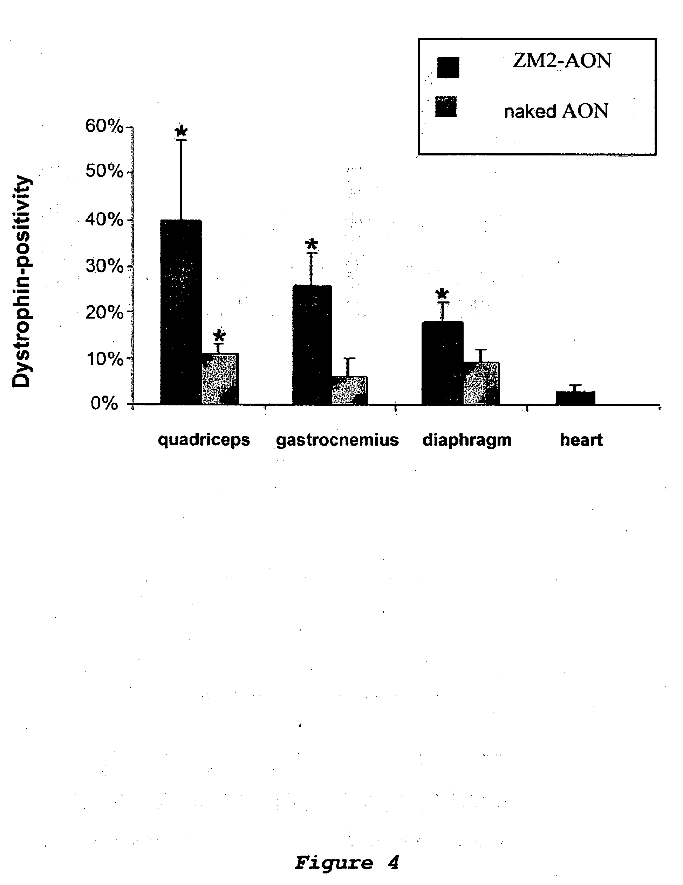

The results from the immunohistochemical quantification, obtained with the manual method, are represented in Figure 4. The histograms represent the percentage of dystrophin-positive fibres in the muscles of mice treated with ZM2/AON(M23D) (black bars) and with naked AON(M23D) (grey bars): At least 5,000 muscle fibres were studied for each muscle The data were analysed by using the Mann-Whitney test and the significance was calculated as compared to untreated *mdx* mice. *: *p*<0.05 statistically significant.

The two used approaches gave very similar results. The dystrophin-positivity percentages in *mdx* mice treated with ZM2/AON(M23D) were 18% (manual method) and 16% (semi-quantitative method) in the diaphragm, 26% and 19% in the gastrocnemius, 40% and 35% in the quadriceps, 3% and 6% in the heart. Dystrophin restoration was found to be significantly higher in mice treated with ZM2/AON(M23D) as compared to those treated with naked AON(M23D). The latter were in fact characterised by a maximum of 11-13% dystrophin-positive fibres in the quadriceps.

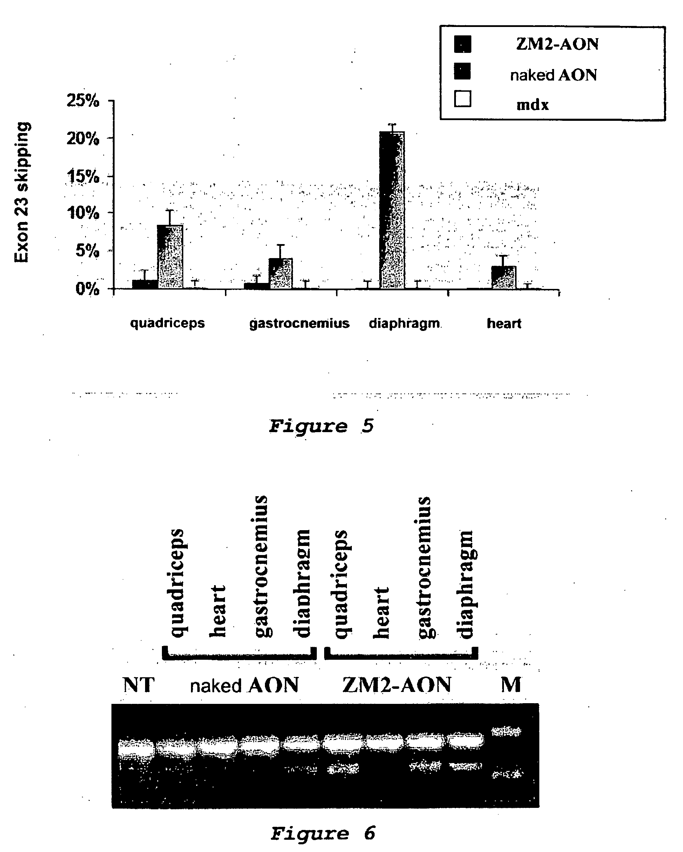

The real time exon-specific RT-PCR assays (ESRA) showed an exon 23 skipping percentage that ranged from 4 to 21% in the skeletal muscles and of about 3% in the heart (Figure 5): twice the % observed with T1/AON(M23D) in the skeletal muscle but not in the heart. In the Figure, the bars represent the percentage of transcript deriving from skipping of exon 23 (2^{-DDCT} values) calibrated on exons 22 and 25, and normalised against WT mice. The black bars relate to mice treated with naked AON(M23D). The grey bars relate to mice treated with ZM2/AON(M23D). The white bars relate to *mdx* mice.

As expected, skipping of exon 23 was confirmed by RT-PCR in all the muscles except for the heart. Figure 6 shows analysis by nested RT-PCR of dystrophin mRNA from quadriceps, diaphragm, gastrocnemius and heart of group 1 and 2 animals, as well as non-treated animals (NT). A full-length dystrophin transcript was detected in all cDNA samples, and a small amount of a shorter transcript, derived from AON-induced skipping of exon 23, was observed in all the skeletal muscles, except for the heart, from animals treated with ZM2/AON(M23D) and in the diaphragm of mice treated with naked AON(M23D). M. 1X molecular weight marker (Roche).

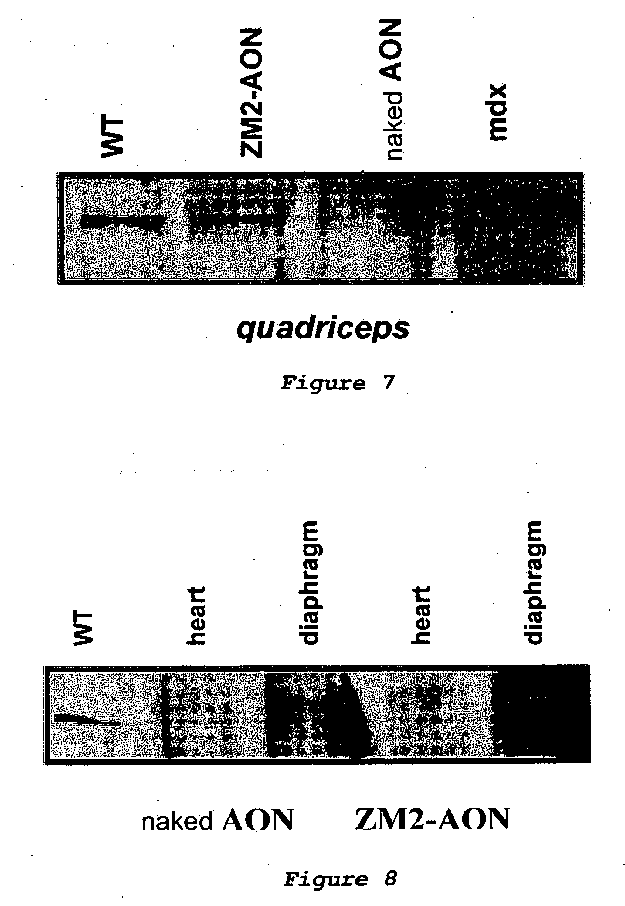

The Western Blotting analysis demonstrated the presence of high molecular weight dystrophin in the skeletal muscles (quadriceps and diaphragm) from *mdx* mice treated with the ZM2/AON(M23D) complex. Figures 7 and 8 represent dystrophin immunoblots obtained with the antibody DYS2. For the WT sample, the total amount of loaded protein was 1/15 of the amount in the other lanes. Figure 7: restored protein expression in the quadriceps of mice treated with ZM2/AON(M23D) and naked AON(M23D). The quadriceps from WT mice and untreated *mdx* mice were used as positive and negative controls. Figure 8: dystrophin is clearly visible in the diaphragm of both mice treated with naked AON(M23D) and mice treated with ZM2/AON(M23D), and is also visible, although at a low intensity, in the heart of mice treated with ZM2/AON(M23D). The diaphragm of WT mice was used as a control.

Therefore, as expected based on the immunohistochemical and transcriptional data, dystrophin is detectable in the heart, even if faintly, by Western blotting. It is interesting to note that a measurable, even if lower, dystrophin restoration was obtained by using the same dose of naked AON. Such a restoration was well visible by immunohistochemical analysis and less visible by Western blotting (Figures 7 and 8). Moreover, the total amount of AON(M23D) injected in the form of a complex with the ZM2 nanoparticles (52.5 mg/kg) was much lower than the dose indicated in previous studies with naked AON (120-600 mg/kg).

Subsequently, it was assessed if dystrophin restoration was visible in the smooth muscles of treated animals, particularly in the arrector pili muscle. Expression of dystrophin in the human arrector pili muscle had been documented previously and was observed to be absent in DMD patients, but had not been yet studied in the *mdx* animal model.

Dystrophin was proven to be actually expressed in the arrector pili muscle of wild-type mice (WT), but absent in *mdx* mice, and *mdx* mice treated with ZM2/AON(M23D) (but not mice treated with the same identical dose of naked AON) clearly presented dystrophin rescue in this smooth muscle.

To that end, skin biopsy sections obtained from the back of the neck of WT mice, *mdx* mice treated with ZM2/AON(M23D) and untreated *mdx* mice, were incubated with an antidystrophin antibody (rabbit polyclonal, Santa Cruz Biotechnology) and with a TRITC-conjugated secondary antibody. All the samples were subjected to double staining with an anti-desmin antibody (goat polyclonal 1:10, Santa Cruz Biotechnology) and incubated with a FITC-conjugated anti-goat secondary antibody (1:100 SIGMA). All the samples were observed with a Nikon Eclipse 80i fluorescence microscope. Dystrophin was clearly visible in the smooth arrector pili muscle of WT mice, absent in untreated *mdx* mice, and restored in treated *mdx* mice. Desmin co-localised with the dystrophin spots and was expressed in all the animals.

The inventors have also confirmed dystrophin expression in the human arrector pili muscle from a healthy subject and the absence thereof in a DMD patient. To that end, 7 pm-thick frozen skin biopsy sections from a healthy human subject and a DMD patient were incubated with the anti-dystrophin antibody (rabbit polyclonal, Santa Cruz) and with the TRITC-conjugated secondary antibody. In order to identify the smooth arrector pili muscle cells, the samples were subjected to double staining with an anti-desmin antibody and were detected with a FITC-conjugated secondary antibody. Dystrophin was intensely expressed at the sarcolemma of the smooth arrector pili muscle cells from the healthy subject, but absent in the DMD patient.

Furthermore, the inventors assessed if other sarcolemma proteins, such as alpha-, beta-, delta- and gamma-sarcoglycans, laminin alpha2 chain, dysferlin and caveolin-3, were expressed in the arrector pili muscle of human skin. All the skin samples were subjected to double staining with an anti-desmin antibody (goat polyclonal, Santa Cruz Biotechnology) and detected with a FITC-conjugated anti-goat antibody. All the sarcoglycans were found to be well expressed and detectable in the control human arrector pili muscle, whereas the other sarcolemma proteins analysed (dysferlin, caveolin-3 and laminin alpha2 chain) are not detectable (data not shown). Even though the expression of sarcoglycans is well documented, especially in the blood vasculature, the expression thereof in the arrector pili muscle had not been reported previously.

### Conclusions

The experiments carried out by the present inventors show that the ZM2 nanoparticles of the invention bind and carry antisense oligonucleotides (AON) very efficiently and are not toxic for the cells, neither *in vitro* nor *in vivo.* Systemic injection of the ZM2/AON(M23D) complex into the *mdx* mouse restores synthesis of the dystrophin protein in the skeletal muscle, allowing for the localisation of the protein in 40% of the muscle fibres. The level of skipping of *mdx* exon 23 is about 10%-20%, as measured by Real Time RT-PCR, and dystrophin restoration was demonstrated both by RT-PCR and Western blotting.

The inventors have further verified that dystrophin restoration also occurs in the smooth arrector pili muscle cells in the skin, which represent a histological structure that is easily accessible. In the *mdx* mouse treated with ZM2/AON(M23D), dystrophin synthesis was proven to be restored in the dorsal arrector pili muscle, whereas it is absent in the untreated animals. This finding opens new prospects, making the smooth arrector pili muscle a potential biomarker for estimating the endpoint of a treatment, also suitable to be used for performing periodic examinations so as to monitor the therapeutic effects of a treatment in DMD patients.

## Claims

1. A nanoparticle of the core-shell type comprising:
a) a core of a polymeric material consisting essentially of poly(methyl methacrylate) (PMMA); and
b) a shell consisting of a plurality of chains of a random copolymer of units of N-isopropyl-acrylamide and units of a reactive methacrylate bearing cationic groups.

2. The nanoparticle according to claim 1, wherein the units of reactive methacrylate bearing cationic groups have the following formula: 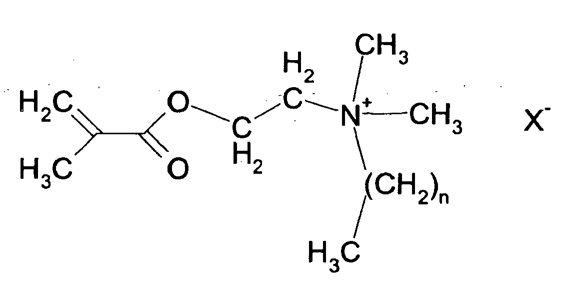 wherein n is an integer comprised between 1 and 10 and X⁻ is a counter ion.

3. The nanoparticle according to claim 2, wherein the counter ion is bromide and n is 7.

4. A nanoparticle/oligonucleotide complex comprising a nanoparticle according to any of claims 1 to 3 and one or more copies of an oligonucleotide associated therewith.

5. The nanoparticle/oligonucleotide complex according to claim 4, wherein said one or more copies of an oligonucleotide are associated to the nanoparticle by adsorption.

6. The nanoparticle/oligonucleotide complex according to claim 5, wherein the oligonucleotide is a therapeutic oligonucleotide, preferably an antisense oligoribonucleotide capable of restoring in all or in part, by exon skipping, the expression of the dystrophin protein within muscle cells.

7. The nanoparticle/oligonucieotide complex according to claim 6, wherein the nucleotide sequence of the antisense oligoribonucleotide consists of SEQ ID NO:1.

8. The nanoparticle/oligonucleotide complex according to any of claims 4 to 7, for use in the therapeutic treatment ofDuchenne muscular dystrophy.

9. The use of a nanoparticle/oligonucleotide complex according to any of claims 4 to 7, for preparing a medicament for the therapeutic treatment of Duchenne muscular dystrophy.

10. The use according to claim 9, wherein the medicament is in a form suitable for administration by injection.

11. A pharmaceutical composition comprising as an active ingredient a nanoparticle/oligonucleotide complex according to any of claims 4 to 7 and a pharmaceutically acceptable carrier.

## Patentansprüche

1. Nanoteilchen des Kern-Schale-Typs, umfassend:
a) einen Kern eines polymeren Materials, bestehend im wesentlichen aus Poly(methylmethacrylat) (PMMA), und
b) eine Schale, bestehend aus einer Vielzahl von Ketten eines statistischen Copolymers von Einheiten von N-Isopropyl-Acrylamid und Einheiten eines reaktiven Methacrylats, welches kationische Gruppen trägt.

2. Nanoteilchen gemäß Anspruch 1, wobei die Einheiten des reaktiven Methacrylats, welches kationische Gruppen trägt, die folgende Formel aufweist: 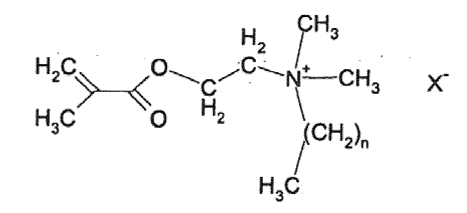 worin n eine ganze Zahl, umfassend zwischen 1 und 10, ist und X- ein Gegenion ist.

3. Nanoteilchen gemäß Anspruch 2, wobei das Gegenion Bromid ist und n 7 ist.

4. Nanoteilchen/Oligonukleotid-Komplex, umfassend ein Nanoteilchen gemäß einem der Ansprüche 1 bis 3 und ein oder mehrere Kopien eines damit gebundenen Oligonukleotids.

5. Nanoteilchen/Oligonukleotid-Komplex gemäß Anspruch 4, wobei die eine oder mehreren Kopien eines Oligonukleotids an das Nanoteilchen durch Adsorption gebunden sind.

6. Nanoteilchen/Oligonukleotid-Komplex gemäß Anspruch 5, wobei das Oligonukleotid ein therapeutisches Oligonukleotid ist, vorzugsweise ein Antisense-Oligoribonukleotid, welches befähigt ist, im Gesamten oder in Teilen, durch Exon-Skipping, die Expression des Dystrophinproteins innerhalb von Muskelzellen wiederherzustellen.

7. Nanoteilchen/Oligonukleotid-Komplex gemäß Anspruch 6, wobei die Nukleotidsequenz des Antisense-Oligoribonukleotids aus SEQ ID Nr. 1 besteht.

8. Nanoteilchen/Oligonukleotid-Komplex gemäß einem der Ansprüche 4 bis 7 zur Verwendung in der therapeutischen Behandlung von Duchenne-muskulärer Dystrophie.

9. Verwendung eines Nanoteilchen/Oligonukleotids-Komplexes gemäß einem der Ansprüche 4 bis 7 zur Herstellung eines Medikaments zur therapeutischen Behandlung von Duchenne-muskulärer Dystrophie.

10. Verwendung gemäß Anspruch 9, wobei das Medikament in einer Form ist, geeignet zur Verabreichung durch Injektion.

11. Pharmazeutische Zusammensetzung, umfassend als ein aktiver Bestandteil einen Nanoteilchen/Oligonukleotid-Komplex gemäß einem der Ansprüche 4 bis 7 und einen pharmazeutisch verträglichen Träger.

## Revendications

1. Nanoparticule de type noyau-coque, comprenant :
a) un noyau de matériau polymère, essentiellement constitué de poly-(méthacrylate de méthyle) (PMMA),
b) et une coque constituée de multiples chaînes d'un copolymère statistique de motifs de N-isopropyl-acrylamide et de motifs d'un méthacrylate réactif, porteurs de groupes cationiques.

2. Nanoparticule conforme à la revendication 1, dans laquelle les motifs de méthacrylate réactif porteurs de groupes cationiques correspondent à la formule suivante : 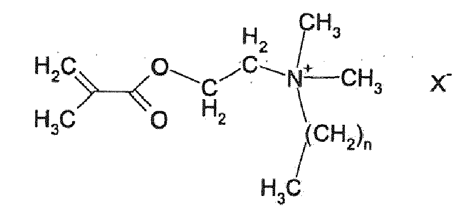 dans laquelle l'indice n est un nombre entier valant de 1 à 10 et X représente un contre-ion.

3. Nanoparticule conforme à la revendication 2, dans laquelle le contre-ion est un ion bromure et l'indice n vaut 7.

4. Complexe de nanoparticule et d'oligonucléotide, comprenant une nanoparticule conforme à l'une des revendications 1 à 3, et une ou plusieurs copies d'un oligonucléotide, associée(s) avec elle.

5. Complexe de nanoparticule et d'oligonucléotide, conforme à la revendication 4, dans lequel ladite ou lesdites copie(s) d'un oligonucléotide est ou sont associée(s) à la nanoparticule par adsorption.

6. Complexe de nanoparticule et d'oligonucléotide, conforme à la revendication 5, dans lequel l'oligonucléotide est un oligonucléotide thérapeutique, de préférence un oligoribonucléotide anti-sens, capable de restaurer, par saut d'exon, tout ou partie de l'expression de la protéine dystrophine au sein des cellules musculaires.

7. Complexe de nanoparticule et d'oligonucléotide, conforme à la revendication 6, dans lequel la séquence de nucléotides de l'oligoribonucléotide anti-sens consiste en la Séquence N° 1.

8. Complexe de nanoparticule et d'oligonucléotide, conforme à l'une des revendications 4 à 7, pour utilisation dans le traitement thérapeutique de la dystrophie musculaire de Duchenne.

9. Utilisation d'un complexe de nanoparticule et d'oligonucléotide, conforme à l'une des revendications 4 à 7, pour la préparation d'un médicament conçu pour le traitement thérapeutique de la dystrophie musculaire de Duchenne.

10. Utilisation conforme à la revendication 9, le médicament devant se présenter sous une forme appropriée pour une administration par injection.

11. Composition pharmaceutique comprenant, comme ingrédient actif, un complexe de nanoparticule et d'oligonucléotide, conforme à l'une des revendications 4 à 7, ainsi qu'un véhicule pharmacologiquement admissible.


### SEQUENCE LISTING

<110> universita degli studi di Ferrara
<120> Nanoparticle of the core-shell type suitable for delivering therapeutic oligonucleotides to target tissues and the use therof for the preparation of a medicament for treating Duchenne muscular dystrophy
<130> PC1011EC
<160> 1
<170> PatentIn version 3.3
<210> 1
   <211> 25
   <212> RNA
   <213> Artificial
<220>
   <223> antisense oligoribonucleotide
<400> 1
   ggccaaaccu cggcuuaccu gaaau 25